(19) Europäisches Patentamt
European Patent Office
Office européen des brevets

(11) Veröffentlichungsnummer: **0 525 686 A1**

# (12) EUROPÄISCHE PATENTANMELDUNG

(21) Anmeldenummer: **92112736.1**

(22) Anmeldetag: **24.07.92**

(51) Int. Cl.[5]: **C07C 235/80**, C07D 277/46, A61K 31/16, A61K 31/425

(30) Priorität: **24.07.91 DE 4124587**

(43) Veröffentlichungstag der Anmeldung: **03.02.93 Patentblatt 93/05**

(84) Benannte Vertragsstaaten: **AT BE CH DE DK ES FR GB GR IT LI LU NL PT SE**

(71) Anmelder: **LUITPOLD PHARMA GmbH**
**Zielstattstrasse 9**
**W-8000 München 70(DE)**

(72) Erfinder: **Strasser, Rupert, Dipl.-Chem., Dr. rer. nat.**
**Römerstrasse 19**
**W-8021 Strasslach(DE)**
Erfinder: **Klauser, Rainer, J., Dipl.-Chem., Dr. rer. nat.**
**Paosostrasse 24**
**W-8000 München 60(DE)**
Erfinder: **Zeiller, Peter, Dr. med. vet.**
**Bellinzonastrasse 8**
**W-8000 München 71(DE)**

(74) Vertreter: **Zumstein, Fritz, Dr.**
**Zumstein & Klingseisen Patentanwälte**
**Bräuhausstrasse 4**
**W-8000 München 2(DE)**

## (54) Dimethylacetessigsäureamide, Verfahren zu ihrer Herstellung und Arzneimittel.

(57) Gegenstand der vorliegenden Erfindung sind neue Dimethylacetessigsäureamid-derivate, Verfahren zu deren Herstellung und sie enthaltende Arzneimittel.

EP 0 525 686 A1

Die Erfindung betrifft Verbindungen der allgemeinen Formel **I**

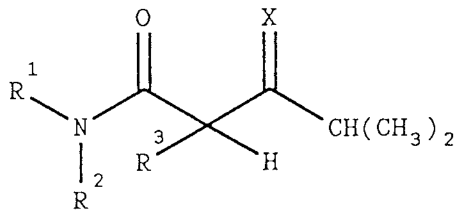

(I)

worin

$R^1$ einen Rest der allgemeinen Formel **II**

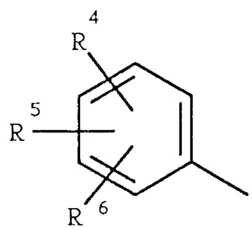

(II)

oder einen **Heteroaryl**rest bedeutet,

**$R^2$** ein Wasserstoffatom oder einen Niedrigalkylrest bedeutet,

**$R^3$** ein Wasserstoffatom, einen Niedrigalkylrest, einen Arylniedrigalkylrest oder einen Rest der allgemeinen Formel **II** bedeutet,

**$R^4$**, **$R^5$** und **$R^6$** unabhängig voneinander Wasserstoffatome, Halogenatome, Hydroxylgruppen, Niedrigalkyloxygruppen, Niedrigalkylgruppen, Nitrogruppen, mit Wasserstoff oder Niedrigalkylgruppen substituierte Aminogruppen, Carbonsäuregruppen, Niedrigalkyloxycarbonylgruppen, Sulfonsäuregruppen oder Trifluormethylgruppen bedeuten,

**X** ein Sauerstoffatom oder eine $=NR^7$-Gruppe bedeutet, wobei

**$R^7$** ein Wasserstoffatom, einen Niedrigalkylrest, einen Rest der allgemeinen Formel **II**, einen Heteroarylrest, einen Hydroxylrest oder einen Niedrigalkyloxyrest bedeutet,

und deren Salze mit physiologisch verträglichen Säuren und Basen,

ausgenommen

N-Phenyl-4-methylpentan-3-on-säureamid,

N-Methyl-N-phenyl-4-methylpentan-3-on-säureamid und

N-(4-Bromphenyl)-4-methyl-2-isopropylpentan-3-on-säureamid.

Die erfindungsgemäßen Verbindungen der allgemeinen Formel **I** liegen im allgemeinen im Sinne einer Keto-Enol-Tautomerie beziehungsweise im Sinne einer Imino-Enamin-Tautomerie einzeln oder im Gemisch vor. Sie können in reiner Form oder als Hydrat vorliegen.

Für die im Zusammenhang mit der vorliegenden Anmeldungsbeschreibung genannten veschiedenen Substituenten bzw. Reste in den Formeln **I** und **II** gelten folgende Erläuterungen:

Beispiele für **Niedrigalkylreste** sind unverzweigte und verzweigte Kohlenwasserstoffreste mit bis zu sechs Kohlenstoffatomen wie Methyl-, Ethyl-, n-Propyl-, iso-Propyl-, n-Butyl-, iso-Butyl-, 1-Methylpropyl-, tert.-Butyl-, n-Pentyl-, 1-Methylbutyl-, 2-Methylbutyl-, 3-Methylbutyl-, 1,1-Dimethylpropyl-, 2,2-Dimethylpropyl-, 1,2-Dimethylpropyl-, 1-Ethylpropyl-, n-Hexyl-, 1-Methylpentyl-, 2-Methylpentyl-, 3-Methylpentyl-, 4-Methylpentyl-, 1,1-Dimethylbutyl-, 2,2-Dimethylbutyl-, 3,3-Dimethylbutyl-, 1,2-Dimethylbutyl-, 2,3-Dimethylbutyl-, 1,3-Dimethylbutyl-, 1-Ethylbutyl-, 2-Ethylbutyl-, 1,1,2-Trimethylpropyl-, 1,2,2-Trimethylpropyl-, 1-Ethyl-2-methylpropyl- oder 1-Ethyl-1-methylpropylreste. Dabei sind Methyl-, Ethyl-, n-Propyl-, iso-Propyl-, n-Butyl- oder iso-Butylreste bevorzugt. Soweit in der vorliegenden Anmeldung der Ausdruck "Niedrigalkyl" allein oder in Kombination mit anderen funktionellen Gruppen (z.B. Niedrigalkyloxy-, Niedrigalkyloxycarbonyl-,Niedrigalkylmercaptogruppen) erscheint, besitzt dieser Ausdruck die vorstehend angegebenen Definitionen.

Beispiele für **Arylniedrigalkylreste** bei **$R^3$** sind die vorstehend definierten Niedrigalkylreste, verknüpft mit einem Rest der allgemeinen Formel **II**, der wie vorstehend definiert ist. Bevorzugte Arylniedrigalkylreste sind Benzyl-, Phenethyl-, 4-Fluorbenzyl-, 2-(4'-Fluorphenyl)ethyl-, 4-Chlorbenzyl-, 2-(4'-Chlorphenyl)-ethyl-

,2-Hydroxybenzyl-, 2-(2'-Hydroxyphenyl)ethyl-, 3-Hydroxybenzyl-, 2-(3'-Hydroxyphenyl)ethyl-, 4-Hydroxybenzyl-, 2-(4'-Hydroxyphenyl)ethyl-, 2-Methoxybenzyl-, 2-(2'-Methoxyphenyl)ethyl-, 4-Methoxybenzyl-, 2-(4'-Methoxyphenyl)ethyl-, 2,5-Dimethoxybenzyl-, 2,4-Dimethoxybenzyl-, 4-Methylbenzyl-, 2-(4'-Methylphenyl)-ethyl-, 4-Nitrobenzyl-, 2-(4'-Nitrophenyl)ethyl-, 4-(N,N-Dimethylamino)benzyl-, 2-(4'-N,N-Dimethylaminophenyl)ethyl, 2-Trifluormethylbenzyl-, 3-Trifluormethylbenzyl-, 4-Trifluormethylbenzyl-, 4-Methoxycarbonylbenzyl-, 4-Methylmercaptobenzyl-, 2-Methoxy-5-methylbenzylreste.

Beispiele für **Halogenatome** sind Fluor-, Chlor-, Brom- und Iodatome.

Beispiele für **Heteroarylreste** sind ein, zwei oder drei gleiche oder unterschiedliche Heteroatome wie Stickstoffatome, Sauerstoffatome oder Schwefelatome enthaltende fünf- und sechsgliedrige monocyclische oder mit Benzol an beliebiger Stelle annellierte aromatische Ringsysteme. Besonders zu nennen sind Furanyl-, Thienyl-, Pyrrolyl-, Pyrazolyl-, Imidazolyl-, Triazolyl-, Thiazolyl-, Oxazolyl-, Isothiazolyl-, Isoxazolyl-, Thiadiazolyl-, Pyridyl-, Pyrimidyl-, Pyrazinyl-, Triazinyl-, Benzofuranyl-, Benzothienyl-, Indolyl-, Benzoxazolyl-, Benzothiazolyl-, Benzimidazolyl-, Chinolinyl- oder Isochinolinylreste. Vorstehend genannte Heteroarylreste können unsubstituiert oder mit Halogenatomen, Hydroxylgruppen, Niedrigalkyloxygruppen, Niedrigalkylgruppen, Nitrogruppen, mit Wasserstoff oder Niedrigalkylgruppen substituierten Aminogruppen, Carbonsäuregruppen, Carbonsäureniedrigalkylestern, Mercaptogruppen, Niedrigalkylmercaptogruppen, Sulfonsäuregruppen oder Trifluormethylgruppen substituiert sein. Bevorzugt sind die folgenden Heteroarylreste: 2-Furanyl-, 2-Thienyl-, 2-Imidazolyl-, 2-Thiazolyl-, 2-Pyridyl- oder 2-Pyrimidylreste. Heteroarylreste können über alle Kohlenstoffatome im Ring mit Verbindungen der allgemeinen Formel **I** verknüpft sein.

Die am Rest der allgemeinen Formel **II** vorhandenen Substituenten $\mathbf{R}^4$, $\mathbf{R}^5$ und $\mathbf{R}^6$ können in verschiedenen Positionen des Phenylrings stehen wie beispielsweise in ortho- und para-, aber auch in meta-Position zur Verknüpfungsstelle des Phenylrings.

Sofern die erfindungsgemäßen Verbindungen in Salzform vorliegen, handelt es sich um die Salze physiologisch verträglicher anorganischer oder organischer Basen und Säuren.

Beispiele für Salze mit physiologisch verträglichen Basen sind Ammonium-, Natrium-, Kalium-, Lithium-, Magnesium- und Calciumsalze, sowie Salze mit Ethanolamin, Triethanolamin, Morpholin oder Piperidin.

Beispiele für Salze mit physiologisch verträglichen Säuren sind Citrat-, Tartrat-, Acetat-, Chlorid- und Bromid-haltige Salze.

Die Verbindungen der allgemeinen Formel **I** können in Analogie zu Methoden, die aus der Literatur und aus verschiedenen Patentschriften bekannt sind, hergestellt werden.

**Methode 1:**

Verbindungen der allgemeinen Formel **I** können aus Isobutyrylessigsäureestern, beispielsweise Isobutyrylessigsäuremethylestern, (Darstellung aus Acetessigsäureestern analog "Organikum", Organisch-chemisches Grundpraktikum, VEB Deutscher Verlag der Wissenschaften, 16. Auflage, Berlin 1986, S. 480 und S. 478) und aromatischen Aminen in hochsiedenden aromatischen Kohlenwasserstoffen bei Temperaturen über 140 °C mit oder ohne Verwendung allgemein üblicher Acylierungskatalysatoren (analog L. Knorr; Ann. Chem. **236**, 69 (1886); J. Roos; Chem. Ber. **21**, 19 (1888); L. Knorr, B. Reuter; Chem. Ber. **27**, 1169 (1894)) hergestellt werden.

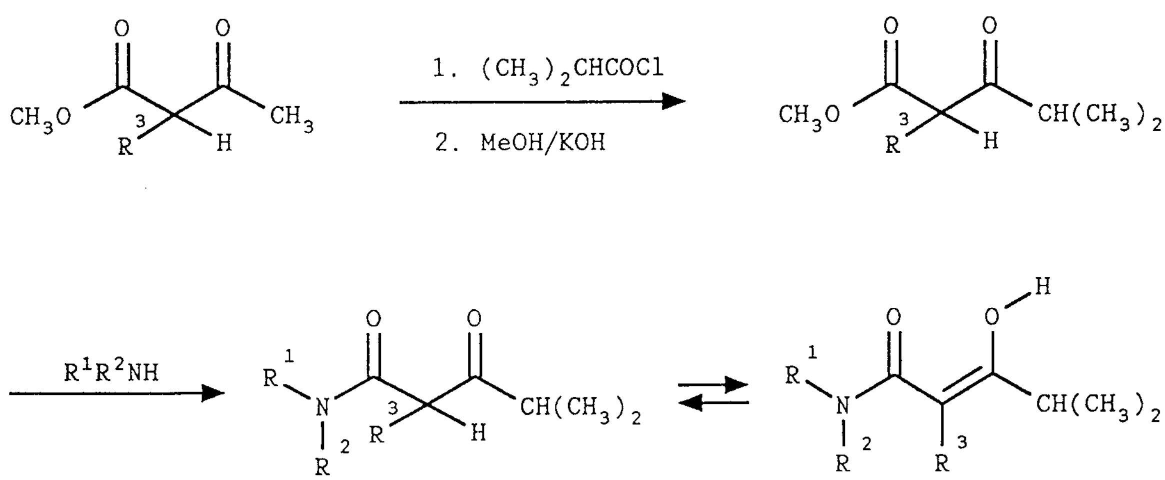

**Methode 2:**

Die Umsetzung von aromatischen Aminen mit Diketen (z.B. Chick, Wilsmore; J. Chem. Soc. **1908**, 946; Boese; Ind. Eng. Chem. **32**, 16 (1940); Williams, Krynitsky; Org. Synth. **Coll. Vol. III;** 10 (1955)) bzw. mit Diketen-Aceton-Addukt (z.B. M. F. Carrol, A. R. Bader; J. Am. Chem. Soc. **75**, 5400 (1953); R. J.Clemens, J. A. Myatt; J. Org. Chem. **50**, 2431 (1985)) liefert ausschließlich Acetessigsäureamide mit $\mathbf{R}^3$ = H.

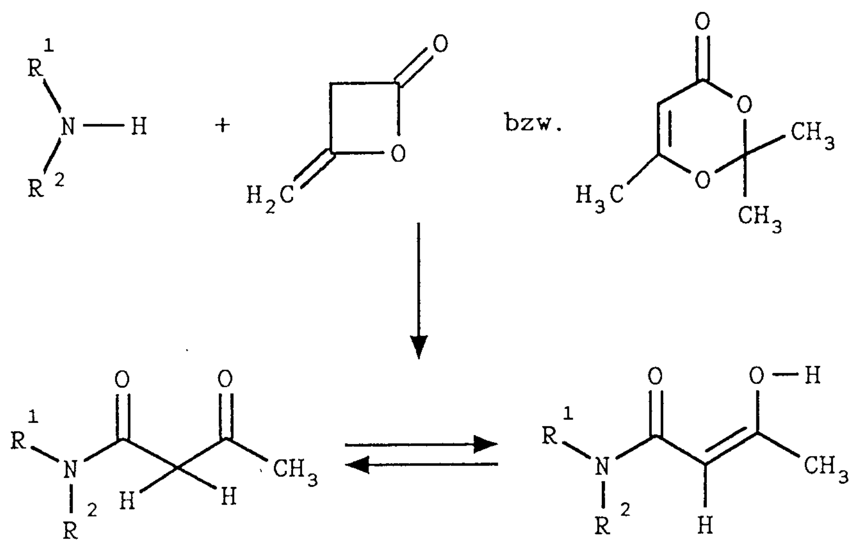


Von dieser Stufe ausgehend gelingt die Umacylierung ebenfalls nach Methode 1 zu den Isobutyrylessigsäureamiden.

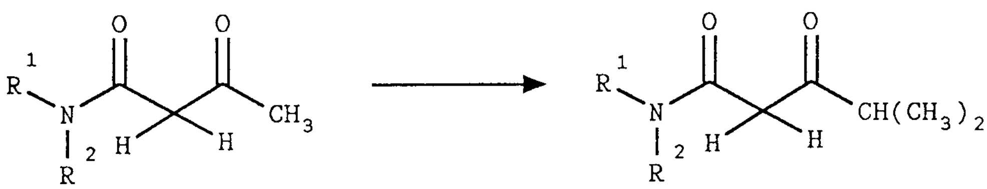

**Methode 3:**

Eine weitere Zugangsmöglichkeit für Verbindungen der allgemeinen Formel **I** liegt in der Umsetzung von α-deprotonierten Carbonylverbindungen mit aromatischen Isocyanaten (analog A. P. Krapcho, W. P. Stephens; J. org. Chem. **45**, 1106 (1980); N. A. LeBel, R. M. Cherluck, E. A. Curtis; Synthesis **11**, 678 (1973)).

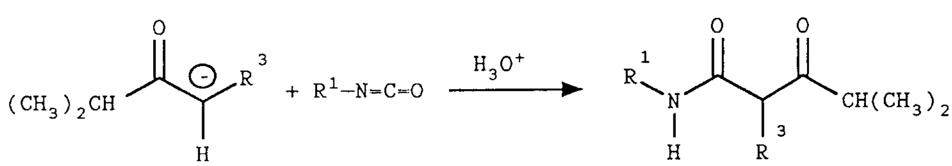


**Methode 4:**

Die Deprotonierung von N-aromatischen Amiden zum entsprechenden Dianion und weitere Umsetzung mit Isobuttersäuremethylester führt ebenfalls zu Verbindungen der allgemeinen Formel **I** (analog I. T. Barnish, C. R. Hauser, J. F. Wolfe; J. Org. Chem. **33**, 2116 (1968)).

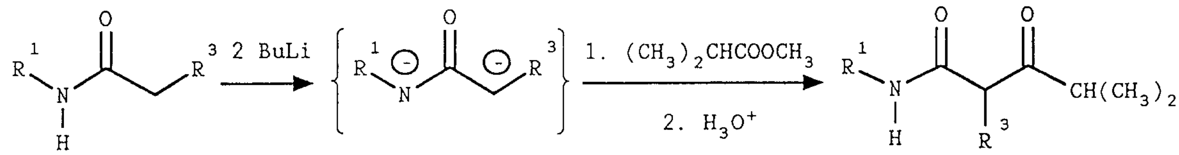


**Methode 5:**

Die Substitution von **R**$^3$ = H gegen **R**$^3$ = Niedrigalkyl bzw. Arylniedrigalkyl gelingt auf der Stufe der Acylessigsäureester nach "Organikum", Organisch-chemisches Grundpraktikum, VEB Deutscher Verlag der Wissenschaften, 16. Auflage, Berlin 1986, S. 517.

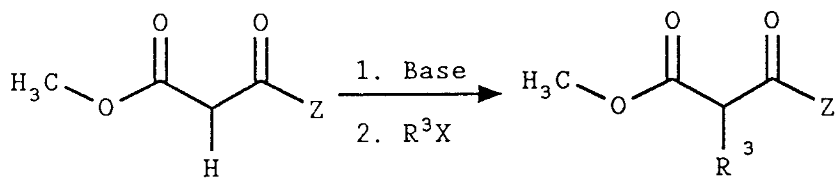


Z = Methyl, iso-Propyl

**Methode 6:**

Acylessigsäureamide lassen sich unter Phasen-Transfer-Katalyse analog P. S. Raman, M. A. Ashrof; Curr. Sci. **48**, 583 (1979) alkylieren.

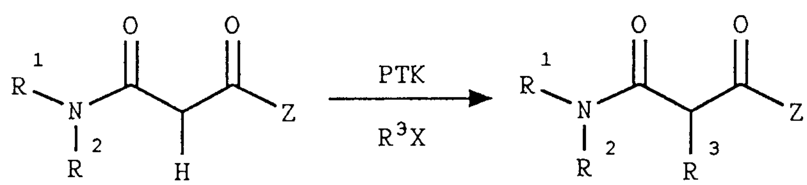


Z = Methyl, iso-Propyl

**Methode 7:**

Kondensation von Acylessigsäureestern mit Aldehyden oder Ketonen nach "Organikum", Organisch-chemisches Grundpraktikum, VEB Deutscher Verlag der Wissenschaften, 16. Auflage, Berlin 1986, S. 459 und anschließende katalytische Hydrierung liefern ebenfalls C-2-substituierte Acylessigsäureester, die nach Methode 1 weiter zur Zielverbindung umgesetzt werden können.

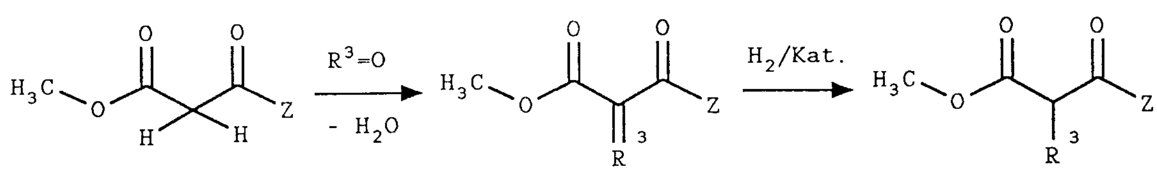


Z = Methyl, iso-Propyl

**Methode 8:**

Beschrieben ist diese Umsetzung auch für Acylessigsäureamide (Z. H. Khalil, A. S. Yanni; J. Indian Chem. Soc. **LVIII**, 168 und 494 (1981)).

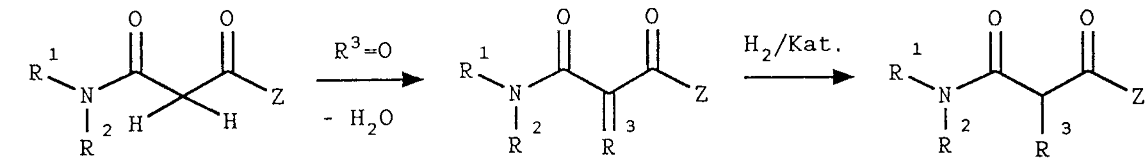


Z = Methyl, iso-Propyl

**Methode 9:**

Analog zu Literaturvorschriften setzen sich Isobutyrylessigsäureamide mit Amoniak oder mit Aminen zu den entsprechenden Enamin- bzw. Iminoverbindungen um (z.B. D. Nardi, A. Tajana, R. Pennini; J. Heterocycl. Chem. **12**, 139 (1979). Mit Hydroxylamin werden Hydroxyiminoverbindungen erhalten (z.B. A. Kettrup, T. Neustadt; Z. Naturforsch. **28b**, 86 (1973), L. Knorr, B. Reuter; chem. Ber. **27**, 1169 (1894)). Niedrigalkyloxyamine liefern Niedrigalkyloxyiminoverbindungen.

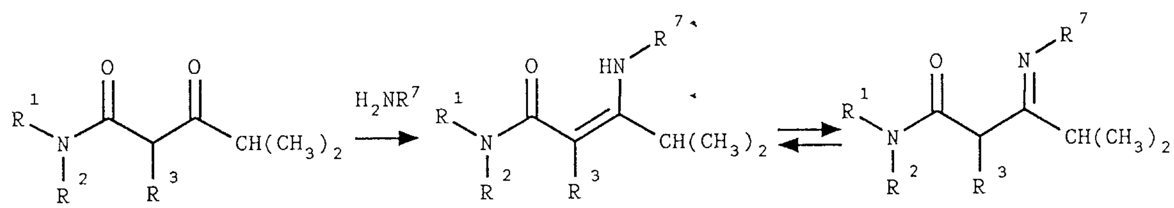


**Methode 10:**

Werden wie in Methode 4 nach Deprotonierung von aromatischen Amiden die entsprechenden Dianionen mit Isobuttersäurenitril anstatt mit Isobuttersäureestern versetzt, führt dies zu Verbindungen der allgemeinen Formel **I** mit **X** = NH (analog I. T. Barnish, C. R. Hauser, J. F. Wolfe; J. Org. Chem. **33**, 2116 (1968)).

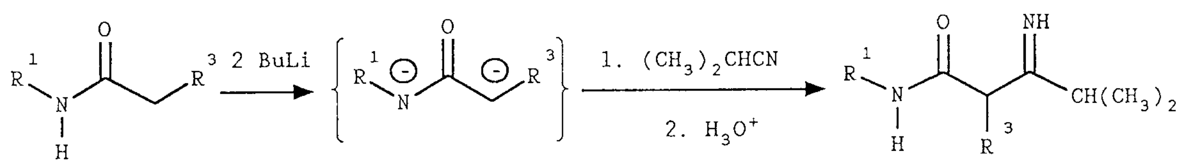


Verbindungen aus der Gruppe der Acylessigsäureamide sind bekannt und finden in der Chemie eine breite Anwendung. Aus der Broschüre "Organische Zwischenprodukte", Wacker-Chemie GmbH, März 1989, Nr. 6.10. ist die Verbindung N-Phenyl-4-methyl-pentan-3-on-säureamid als Zwischenprodukt bekannt. Ferner ist sie auch in J. Med. Chem. **33**, 61 (1990) als Zwischenprodukt beschrieben. Die Verbindung N-Methyl-N-phenyl-4-methylpentan-3-on-säureamid soll als Zwischenprodukt in der BE-634.930 (bei der Herstellung des dort beschriebenen Endprodukts Nr.15) auftreten. Aus J. Org. Chem. USSR (Engl. Transl.) **14**, 1069 - 1079 (1978) ist schließlich die Verbindung N-(4-Bromphenyl)-4-methyl-2-isopropylpentan-3-on-säureamid bekannt. Für keine der drei genannten Verbindungen wurden pharmakologische Wirkungen beschrieben. Sonstige Verbindungen der allgemeinen Formel **I** sind nicht bekannt geworden.

Pharmakologische Wirkungen von Verbindungen aus der Gruppe der Acylessigsäureamide sind bisher ebenfalls nicht bekannt geworden.

Die in US-4.256.759 und EP-A-372.470 beschriebenen Wirksubstanzen stehen nur bezüglich eines Teils ihres Grundgerüsts in struktureller Nähe zu den erfindungsgemäßen Verbindungen der allgemeinen Formel **I**. Diese $\alpha$-Cyano-$\beta$-oxo-propionsäureamidderivate sind wegen ihrer obligatorisch vorgeschriebenen Cyanogruppe und wegen ihres in $\beta$-Stellung obligatorisch vorgeschriebenen heteroaromatischen Restes nicht

vergleichbar mit den Verbindungen der vorliegenden Erfindung.

Im Zusammenhang mit der vorliegenden Erfindung wurde überraschend gefunden, daß Substanzen der allgemeinen Formel **I** wertvolle pharmakologische Eigenschaften aufweisen und insbesondere als antientzündliche Wirkstoffe eingesetzt werden können. Neben der guten Verträglichkeit - die $LD_{50}$ der Verbindung aus Beispiel 1 liegt beispielsweise bei über 1000 mg/kg - zeichnen sich Verbindungen der allgemeinen Formel **I** durch nützliche analgetische und antipyretische Eigenschaften aus. Insbesondere eignen sich die erfindungsgemäßen Verbindungen zur Behandlung entzündlicher Erkrankungen bei Mensch und Tier. Die Krankheitsbilder, die mit den erfindungsgemäßen Verbindungen behandelt werden können, umfassen alle entzündlichen Zustände, wie zum Beispiel Entzündungen der Haut wie Radiodermatitis, Psoriasis, Akne, Ekzeme, Entzündungen am Auge wie zum Beispiel Conjunktivitis, Entzündungen im Hals-Nasen-Ohren-Bereichwie zum Beispiel katarralische Infektionen der oberen Luftwege und Sinusitis, entzündliche Erkrankungen des Gefäßsystems wie Thrombophlebitis oder Vaskulitis und Entzündungen des Nervensystems wie zum Beispiel Neuralgien oder Neuritiden. Bevorzugt zu behandelnde entzündliche Erkrankungen sind Entzündungen im Bereich des Bewegungsapparates wie Arthritiden, Arthrose, Tendinitis, Tendopathien, Kontusionen und Distorsionen, Weichteilrheumatismus, Gicht und Lumbalgien. Die erfindungsgemässen Verbindungen eignen sich auch zur Behandlung von bestimmten Erkrankungen oder Störungen, bei denen der Schmerz im Vordergrung steht. Hier sind postoperative Schmerzzustände, Schmerzen nach Zahnextraktion und Migräne zu nennen.

Die Prüfung auf **antientzündliche Wirkung** (C. A. Winter, E. A. Risley, G. W. Nuss; Proc. Soc. Exp. Biol. Med. **111**, 544 (1962)) erfolgte durch mit 0.5 %iger wäßriger Carrageenin-Lösung erzeugte Ödeme der rechten Hinterpfote an Gruppen mit je zehn nüchternen, ca. 150 g schweren weiblichen Wistar-Ratten. Dazu wurden mit einem Plethysmometer die Volumina der markierten rechten Hinterpfote unmittelbar vor und drei Stunden nach der Injektion der Carrageenin-Lösung gemessen. Die zu prüfenden Substanzen wurden 30 Minuten vor Erzeugung des Ödems als Gummi arabicum Suspension mittels einer Schlundsonde p.o. verabreicht. Die Kontrollgruppe erhielt reines Leitungswasser. Die Einzeldosis pro Tier lag bei 200 mg/kg Körpergewicht.

Zur Auswertung wurde die prozentuale Zunahme des Pfotenvolumens für jedes Tier nach folgender Formel berechnet:

$$\frac{\text{Volumen 2. Messung x 100}}{\text{Volumen 1. Messung}} - 100 = \text{Differenz (\%)}$$

dann wurden Mittelwerte und Streuung für alle so berechneten Werte einer Gruppe bestimmt. Der Unterschied zwischen der mit Prüfsubstanz behandelten Versuchs- und der zugehörigen unbehandelten Gruppe wurde nach der Formel

$$\frac{\text{MW Versuch (Testsubstanz) x 100}}{\text{MW Kontrolle}} - 100 = \text{\% Hemmung}$$

ermittelt. Die statistische Auswertung erfolgte nach den üblichen Verfahren.

**Tabelle 1** zeigt an einigen Beispielen die gemessene antientzündliche Wirkung.

TABELLE 1

| Verbindung aus Beispiel | % Hemmung |
|---|---|
| 1 | 52 |
| 2 | 32 |
| 3 | 27 |
| 4 | 26 |

Die erfindungsgemäßen Verbindungen können in einer Vielzahl pharmazeutischer Zubereitungsformen und Dosierungen appliziert werden, wie zum Beispiel Tabletten, Dragees, Kapseln, flüssige oral einzunehmende Zubereitungen, Salben, Gele, Pflaster, Injektionslösungen oder Sprays, wobei allgemein übliche Träger- und Hilfsstoffe verwendet werden können, die mit den erfindungsgemäßen Verbindungen verträglich sind. Die Dosierungen können 10 bis 500 mg einer Verbindung der allgemeinen Formel **I** oder ihres Salzes pro Dosiseinheit betragen, bei der Verabreichung von ein bis vier Dosiseinheiten pro Tag.

Gegenstand der vorliegenden Erfindung sind auch pharmazeutische Zubereitungen (Arzneimittel), die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Bevorzugte pharmazeutische Zubereitungen sind Tabletten, Dragees, Kapseln, Pillen, Granulate, Suppositorien, Lösungen, Suspensionen und Emulsionen, Pasten, Salben, Gele, Cremes, Lotions, Puder, Sprays und Aerosole.

Tabletten, Dragees, Kapseln, Pillen und Granulate können den oder die Wirkstoffe neben den üblichen Trägerstoffen enthalten. Dazu gehören a) Füll- und Streckmittel, zum Beispiel Stärken, Milchzucker, Rohrzucker, Glucose, Mannit und Kieselsäure, b) Bindemittel, zum Beispiel Carboxymethylcellulose, Alginate, Gelatine, Polyvinylpyrrolidon, c) Feuchthaltemittel, zum Beispiel Glycerin, d) Sprengmittel, zum Beispiel Agar-Agar, Calciumcarbonat und Natriumbicarbonat, e) Lösungsverzögerer, zum Beispiel Paraffin, f) Resorptionsbeschleuniger, g) Netzmittel, zum Beispiel Cetylalkohol, Glycerinmonostearat, h) Adsorptionsmittel, zum Beispiel Kaolin und Bentonit und i) Gleitmittel, zum Beispiel Talkum, Calcium- und Magnesiumstearat und feste Polyethylenglycole oder Gemische der unter a) bis i) aufgeführten Stoffe.

Die Tabletten, Dragees, Kapseln, Pillen und Granulate können mit üblichen gegebenenfalls Opalisierungsmittel enthaltenden Überzügen, zum Beispiel Zucker, Überzugslacken, versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen zum Beispiel Polymersubstanzen und Wachse verwendet werden können.

Der oder die Wirkstoffe können gegebenenfalls mit einem oder mehreren der eben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel $C_{14}$-Alkohol mit $C_{16}$-Fettsäure oder Gemische dieser Stoffe).

Cremes enthalten neben dem oder den Wirkstoffen als ölige Grundlage in erster Linie Fettalkohole, zum Beispiel Lauryl-, Cetyl-, Stearylalkohol, Fettsäuren, zum Beispiel Palmitin- oder Stearinsäure, flüssige bis feste Wachse, zum Beispiel Isopropylmyristat, Wollwachse oder Bienenwachs und/oder Kohlenwasserstoffe, zum Beispiel Vaseline (Petrolatum) oder Paraffinöl. Als Emulgatoren verwendet man bevorzugt solche mit vorwiegend hydrophilen Eigenschaften, zum Beispiel nichtionische Emulgatoren wie Fettsäureester von Polyalkoholen, Ethylenoxidaddukte davon wie Polyglycerinfettsäureester oder Polyoxyethylensorbitanfettsäureester (Tweens) oder ionische Emulgatoren wie Alkalimetallsalze von Fettalkoholsulfaten, zum Beispiel Natriumlaurylsulfat, Natriumcetylsulfat oder Natriumstearylsulfat. Zur Wasserphase können Mittel zugesetzt werden, welche die Austrocknung der Creme verhindern, zum Beispiel Polyalkohole wie Glycerin, Sorbit, Propylenglycol und/oder Polyethylenglycole.

Für Salben kommen neben dem oder den Wirkstoffen als Trägerstoffe in erster Linie Kohlenwasserstoffe, zum Beispiel Vaseline oder Paraffinöl in Frage, die zur Verbesserung des Wasserbindungsvermögens vorzugsweise geeignete Fettalkohole oder Ester davon, zum Beispiel Cetylalkohol oder Wollwachse enthalten. Emulgatoren sind entsprechende lipophile Substanzen wie Sorbitanfettsäureester. Zur Wasserphase können Feuchthaltungsmittel wie zum Beispiel Glycerin oder Propylenglycol zugesetzt werden.

Sprays und Puder können neben dem oder den Wirkstoffen die üblichen Trägerstoffe enthalten, zum Beispiel Milchzucker, Talkum, Kieselsäure, Aluminiumhydroxid, Calciumsilicat und Polyamidpulver oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, zum Beispiel Wasser, Ethanol, Isopropanol, Ethylcarbonat, Benzylalkohol, Benzylbenzoat, Propylenglycol, 1,3-Butylenglycol, Öle, insbesondere Baumwollsaatöl, Erdnußöl, Maisöl, Ceshewnußöl, Ricinusöl und Sesamöl, Glycerin, Glycerinformal, Polyethylenglycole und Fettsäureester des Sorbitans oder Gemische dieser Stoffe enthalten.

Suspensionen können neben dem oder den Wirkstoffen die üblichen Trägerstoffe wie flüssige Verdünnungsmittel, zum Beispiel Ethanol, Propylenglycol, Suspendiermittel, zum Beispiel ethoxylierte Isostearylalkohole, Polyoxyethylensorbit- und Sorbitanester, mikrokristalline Cellulose, Aluminiummetahydroxid, Bentonit, Agar-Agar oder Traganth oder Gemische dieser Stoffe enthalten.

Die genannten Formulierungsformen können auch Färbemittel, Konservierungsstoffe sowie geruchs-und geschmacksverbessernde Zusätze, zum Beispiel Pfefferminzöl und Eucalyptusöl und Süßmittel, zum Beispiel Saccharin enthalten.

Die therapeutisch wirksamen Verbindungen sollen in den oben aufgeführten pharmazeutischen Zubereitungen vorzugsweise in einer Konzentration von etwa 0.1 bis 99.5, vorzugsweise von etwa 0.5 bis 95 Masse-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, zum Beispiel durch Mischen der Wirkstoffe mit den Trägersoffen.

Die vorliegenden Wirkstoffe bzw. pharmazeutischen Zubereitungen, die einen oder mehrere Wirkstoffe enthalten, können in der Human- und Veterinärmedizin zur Verhütung, Besserung und/oder Heilung entzündlicher Krankheiten eingesetzt werden.

Im allgemeinen erweist es sich als vorteilhaft, in der Humanmedizin den oder die erfindungsgemäßen Wirkstoffe in Gesamtdosen von etwa 10 bis etwa 500 mg, vorzugsweise 25 bis 500 mg pro Dosiseinheit bei Verabreichung von ein bis vier Dosiseinheiten pro Tag zur Erzielung der gewünschten Ergebnisse zu applizieren.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genennten Wirkstoffmenge auszukommen, während in anderen Fällen die oben angeführte Menge Wirkstoff überschritten werden muß.

Die nachfolgenden Beispiele erläutern die Erfindung näher, ohne ihren Umfang darauf zu beschränken.

## Beispiele

### Beispiel 1:

**N-(2-Methoxyphenyl) 4-methylpentan-3-on-säureamid**

Eine Lösung aus 10 g (69.9 mmol) Isobutyrylessigsäuremethylester, 7.82 ml (69.4 mmol) o-Anisidin und einigen Tropfen Diethanolamin in 50 ml Xylol wird solange bei 135 - 150 °C erhitzt, bis kein bei der Reaktion freiwerdendes Methanol mehr überdestilliert. Die Lösung wird nach dem Abkühlen mit verdünnter Natronlauge mehrmals extrahiert, die alkalische Lösung mit verdünnter Säure angesäuert und mit Essigester rückextrahiert. Waschen mit verdünnter Kochsalzlösung, Trocknen über Natriumsulfat und Abziehen des Lösungsmittels liefert ein öliges Rohprodukt. Nach Chromatographie (Diisopropylether/Essigester = 1/1) erhält man 9.39 g (58 %) der Titelverbindung als sauberes und öliges Produkt.

| Elementaranalyse: | | | |
|---|---|---|---|
| | % C | % H | % N |
| berechnet: | 66.35 | 7.30 | 5.95 |
| gefunden : | 66.31 | 7.25 | 6.09 |

MS: 235 ($M^{+\cdot}$), 165, 149, 123 (100%), 108, 92, 80, 71, 65, 52.

IR (KBr): 3300, 2965, 1710, 1680, 1643, 1600, 1535, 1487, 1460, 1435, 1335, 1290, 1253, 1220, 1176, 1117, 1045, 1028, 750 $cm^{-1}$.

$^1$H-HMR ($d_6$-Aceton): $\delta$ = 1.12 (d, 6H, 2 x $CH_3$), 2.83 (dq, 1H, -$CH(CH_3)_2$), 3.73 (s, 2H, -$CH_2$-), 3.84 und 3.87 (2s, 3H, $OCH_3$), 5.43 (s, 1H, =CH-), 6.76 - 7.08 (m, 3H, Ar-H), 8.15 - 8.45 (m, 1H, Ar-H), 9.23 (s-breit, 1H, NH), 13.88 (s, 1H, Enol-OH); Keto : Enol = 4 : 1.

### Beispiel 2:

**N-(2-Thiazolyl)-4-methylpentan-3-on-säureamid**

Eine Lösung aus 20 g (138.7 mmol) Isobutyrylessigsäuremethylester und 13.9 g (138.7 mmol) 2-Aminothiazol werden in Xylol bei 165 °C Ölbadtemperatur solange erhitzt, bis kein Methanol mehr freigesetzt wird. Das abgekühlte Reaktionsgemisch wird mit verdünnter Natronlauge mehrmals extrahiert, die alkalische Lösung mit verdünnter Säure angesäuert und mit Essigester rückextrahiert. Waschen mit verdünnter Kochsalzlösung, Trocknen über Natriumsulfat und Abziehen des Lösungsmittels liefert ein dunkelbraunes Öl, das zuerst mit Diisopropylether/Essigester = 1/1 über Kieselgel chromatographiert und anschließend in Diisopropylether umkristallisiert wird. Man erhält so die Titelverbindung in 33 % Ausbeute als farblose Kristalle mit Schmp.: 114 - 115 ° C.

| Elementaranalyse: | | | | |
|---|---|---|---|---|
| | % C | % H | % N | % S |
| berechnet: | 50.92 | 5.71 | 13.20 | 15.11 |
| gefunden : | 50.99 | 5.71 | 13.14 | 14.98 |

MS: 212 ($M^{+\cdot}$), 169, 127, 113, 100 (100%), 71, 58.

IR (KBr): 3120, 2900, 1615, 1550, 1400, 1310, 1270, 1235, 1210, 1185, 1157, 1135, 1005, 920, 790, 778, 748, 720 $cm^{-1}$.

$^1$H-NMR ($d_6$-DMSO): $\delta$ = 1.05 und 1.08 (d, 6H, 2 x $CH_3$), 2.73 (dq, 1H, -$CH(CH_3)_2$), 3.76 (s, 2H, -$CH_2$-), 5.38 (s, 1H, =CH-), 7.23 (d, 1H, Ar-H), 7.48 (d, 1H, Ar-H), 12.14 (s-breit, 1H, Keto-NH), 13.09 (s-breit, 1H, Enol-NH); Keto : Enol = 6.5 : 1.

**Beispiel 3:**

**N-(4-Methoxyphenyl)-4-methylpentan-3-on-säureamid**

Die Darstellung erfolgt analog Beispiel 1: Aus 20 g (139 mmol) Isobutyrylessigsäuremethylester und 1 eg para-Anisidin wird in 30 % Ausbeute die Titelverbindung in Form farbloser Kristalle mit Schmp.: 40 - 41 ° C erhalten.

IR (KBr): 3300, 2970, 1725, 1650, 1605, 1545, 1510, 1465, 1410, 1340, 1300, 1248, 1176, 1109, 1030, 822 $cm^{-1}$.

$^1$H-NMR ($d_6$-Aceton): $\delta$ = 1.08 (d, 6H, 2 x $CH_3$), 2.83 (dg, 1H, -$CH(CH_3)_2$, 3.59 (s, 2H, -$CH_2$-), 3.76 (s, 3H, $OCH_3$), 5.15 (s, 1H, =CH-), 6.73 - 7.69 (m, 4H, Ar-H), 9.16 (s-breit, 1H, NH); Keto : Enol = 3.5 : 1.

**Beispiel 4:**

**N-(2-Methoxy-5-methylphenyl)-4-methylpentan-3-on-säureamid**

Die Darstellung erfolgt analog Beispiel 1: Aus 20 g (139 mmol) Isobutyrylessigsäuremethylester und 1 eg 2-Methoxy-5-methylanilin wird in 28 % Ausbeute die Titelverbindung in Form beigefarbener Kristalle mit Schmp.: 50 - 51 ° C erhalten.

IR (KBr): 3280, 2960, 1703, 1678, 1595, 1545, 1485, 1467, 1428, 1382, 1350, 1329, 1285, 1265, 1225, 1130, 1030, 908, 883, 795, 768, 714 $cm^{-1}$.

$^1$H-NMR ($d_6$-Aceton): $\delta$ = 1.13 (d, 6H, 2 x $CH_3$), 2.26 (s, 3H, Ar-$CH_3$), 2.81 (dq, 1H -$CH(CH_3)_2$, 3.73 (s, 2H, -$CH_2$-), 3.82 und 3.86 (2s, 3H, $OCH_3$), 5.41 (s, 1H, =CH-), 6.87 (s, 2H, Ar-H), 8.20 (s, 1H, Ar-H), 9.15 (s-breit, 1H, NH); Keto : Enol = 4.2 : 1.

**Beispiel 5:**

**Rezeptur zur Herstellung von Tabletten:**

1000 Tabletten stellt man aus den nachstehenden Verbindungen auf die unten beschriebene Weise her. Eine Tablette enthält dann als Wirkstoff 100 mg N-(2-Methoxyphenyl)-4-methylpentan-3-on-säureamid.

| | |
|---|---|
| 1. N-(2-Methoxyphenyl)-4-methylpentan3-on-säureamid | 100 g |
| 2. Milchzucker | 263 g |
| 3. mikrokristalline Cellulose | 120 g |
| 4. Maisstärke | 60 g |
| 5. Magnesiumstearat | 7 g |

Man mischt 1) und 2), mischt 3) und 4) unter, versetzt zuletzt mit 5), mischt und verpreßt direkt.

**Beispiel 6:**

**Rezeptur zur Herstellung einer Creme:**

Die folgende Rezeptur ergibt eine 5 % N-(2-Methoxyphenyl)-4-methylpentan-3-on-säureamid-Creme (Stoffangaben in Gew.-%):

| | |
|---|---|
| N-(2-Methoxyphenyl)-4-methylpentan-3-on-säureamid | 5,00 |
| Emulgator (Gemisch aus Natriumglycerylmonostearat, Natriumstearylsulfat und Natriumcetylsulfat) | 10,00 |
| Mittelkettige Triglyceride | 6,25 |
| Myristylalkohol | 5,00 |
| POE-12-Cetylstearylalkohol | 3,00 |
| Konservierungsmittel | q.s. |
| Wasser | ad 100,00 |

**Patentansprüche**

**1.** . Verbindungen der allgemeinen Formel **I**

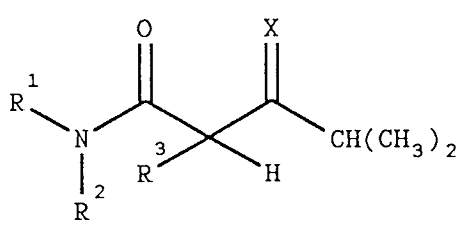

(I)

worin
**R**$^1$ einen Rest der allgemeinen Formel **II**

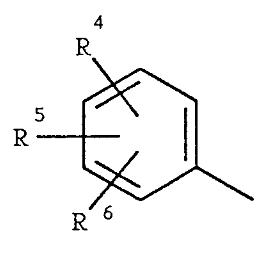

(II)

oder einen **Heteroaryl**rest bedeutet,

**R**$^2$ ein Wasserstoffatom oder einen Niedrigalkylrest bedeutet,

**R**$^3$ ein Wasserstoffatom, einen Niedrigalkylrest, einen Arylniedrigalkylrest oder einen Rest der allgemeinen Formel **II** bedeutet,

**$R^4$**, **$R^5$** und **$R^6$** unabhängig voneinander Wasserstoffatome, Halogenatome, Hydroxylgruppen, Niedrigalkyloxygruppen, Niedrigalkylgruppen, Nitrogruppen, mit Wasserstoff oder Niedrigalkylgruppen substituierte Aminogruppen, Carbonsäuregruppen, Niedrigalkyloxycarbonylgruppen, Mercaptogruppen, Niedrigalkylmercaptogruppen, Sulfonsäuregruppen oder Trifluormethylgruppen bedeuten,

**X** ein Sauerstoffatom oder eine $=NR^7$-Gruppe bedeutet, wobei

**$R^7$** ein Wasserstoffatom, einen Niedrigalkylrest, einen Rest der allgemeinen Formel **II**, einen Heteroarylrest, einen Hydroxylrest oder einen Niedrigalkyloxyrest bedeutet,

und deren Salze mit physiologisch verträglichen Säuren und Basen,

ausgenommen

N-Phenyl-4-methylpentan-3-on-säureamid,
N-Methyl-N-phenyl-4-methylpentan-3-on-säureamid und
N-(4-Bromphenyl)-4-methyl-2-isopropylpentan-3-on-säureamid.

**2.** . Verbindungen gemäß Anspruch **1**, worin **X** die Bedeutung Sauerstoffatom besitzt.

**3.** . Verfahren zur Herstellung von Verbindungen gemäß Anspruch 1, dadurch gekennzeichnet, daß man in an sich bekannter Weise in C-2-Position den Rest **$R^3$** enthaltende Isobutyrylessigsäureester mit den entsprechenden aromatischen Aminen umsetzt und gegebenenfalls die erhaltenen Isobutyrylessigsäureamide mit Ammoniak, Aminen, Hydroxylamin oder Niedrigalkyloxyaminen in die entsprechenden 3-Iminoverbindungen (**X** in der Bedeutung von $=NR^7$) überführt.

**4.** . Arzneimittel für die Verwendung bei Mensch und Tier, bestehend aus, oder enthaltend eine oder mehrere Verbindungen gemäß Anspruch **1**.

Europäisches Patentamt

# EUROPÄISCHER RECHERCHENBERICHT

Nummer der Anmeldung

EP 92 11 2736

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 701 517 (DR. KARL THOMAE GMBH)<br>* Seite 8, Zeile 15 - Seite 9, Zeile 46; Ansprüche 1,5,8; Beispiele 7,18 *<br>--- | 1-4 | C07C235/80<br>C07D277/46<br>A61K31/16<br>A61K31/425 |
| A,D | EP-A-0 372 470 (CIBA-GEIGY AG)<br>* Seite 2, Zeile 24 - Zeile 27; Ansprüche 1,14 *<br>----- | 1,4 | |
| | | | **RECHERCHIERTE SACHGEBIETE (Int. Cl.5)** |
| | | | C07C<br>C07D |

Der vorliegende Recherchenbericht wurde für alle Patentansprüche erstellt

| Recherchenort | Abschlußdatum der Recherche | Prüfer |
|---|---|---|
| DEN HAAG | 28 OKTOBER 1992 | SEUFERT G.H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument